# FASCICULE DE BREVET EUROPEEN

(11) **EP 0 343 186 B1**
(45) Date de publication et mention de la délivrance du brevet: **19.05.1993**
(21) Numéro de dépôt: 88901854.5
(22) Date de dépôt: 09.02.1988
(51) Int. Cl.: C07K 7/08, A61K 39/015, C12N 15/00, G01N 33/569

(54) **MOLECULES COMPORTANT AU MOINS UNE SEQUENCE PEPTIDIQUE PORTEUSE D'UN, OU PLUSIEURS, EPITOPES CARACTERISTIQUES D'UNE PROTEINE PRODUITE PAR $i(P. FALCIPARUM) DANS LES HEPATOCYTES ET COMPOSITIONS LES CONTENANT**
MOLEKÜLE MIT MINDESTENS EINER PEPTIDSEQUENZ EINER ODER MEHRERER EPITOPEN EINES P.FALCIPARUM-PRODUZIERTEN PROTEINS IN HEPATOZYTEN UND ZUSAMMENSETZUNGEN DAVON
MOLECULES COMPRISING AT LEAST ONE PEPTIDIC SEQUENCE CARRYING ONE OR A PLURALITY OF EPITOPES CHARACTERIZING A PROTEIN PRODUCED BY $i(P. FALCIPARUM) IN HEPATOCYTES AND COMPOSITIONS CONTAINING THEM

(30) Priorité: 09.02.1987 FR 8701543
(43) Date de publication de la demande: 29.11.1989
(73) Titulaire: INSTITUT PASTEUR, 75724 Paris Cédex 15 (FR); CENTRE NATIONAL DE LA RECHERCHE SCIENTIFIQUE (CNRS), 75700 Paris Cedex 07 (FR)
(72) Inventeur: MARCHAND, Claudine, F-75012 Paris (FR); DRUILHE, Pierre, F-94160 Saint-Mande (FR); PUIJALON-MERCEREAU, Odile, F-92130 Issy-les-Moulineaux (FR); LANGSLEY, Gordon, F-75006 Paris (FR)
(74) Mandataire: Gutmann, Ernest
(86) Numéro de dépôt international: FR8800074
(87) Numéro de publication internationale: WO8805785

(56) Documents cités:
- WO-A-40/2917
- Biological Abstracts, vol. 78, No. 8, 1984, (Philadelphia, US), P. Druilhe et al.: "Species-specific and stage-specific antigens in exoerythrocytic stages of Plasmadium falciparum", see page 6756, abstract 59971, & Am. J. Trop. Med. Hyg. 33(3): 336-341, 1984 (cited in the application)
- Biological Abstracts, vol. 67, No. 8, (Phlladelphia, US), Q.Z. Hussain et al.: "Vaccine against malaria in rodents: A preliminary communication", see page 4780, abstract 47958, & Indian J. Medl. Res. 64(12), 1836-1840, 1976
- Bilogical Abstracts, vol. 67, No. 8, (Phliadelphia, US), Q.Z.: HUSSAIN et al.: "Vaccine against simian malaria (P. cynomolgi): A preliminary communication", see page 4780, abstract 47957, & Indian J. Med. Res. 64 (12): 1841-1843, 1976
- Science, vol. 234, 12 December 1986, L.H. Miller et al.: "Research twoard malaria vaccines", page 1349-1356
- Nature, vol. 329, No. 6135, 10-16 September 1987, (Basingstoke, Hants., GB), C. Guerin-Marchand et al.: "A liver-stage-specific antigen of Plasmodium falciparum characterized by gene cloning", pages 264-267, see the whole document

## Description

Les parasites responsables du paludisme chez l'homme, dont notamment Plasmodium falciparum ou Plasmodium vivax pour ne citer que les principaux d'entre eux, présentent chez l'hôte humain des morphologies différentes et expriment des antigènes différents en fonction de leur localisation dans l'organisme de l'hôte infecté. Les différences morphologiques et antigéniques de ces parasites au cours de leurs cycles de vie chez l'homme, permettent de définir au moins quatre stades de développement distincts.

Le tout premier stade de développement du parasite chez l'homme correspond à la forme sporozoïte introduite dans le sang de l'hôte, par piqûres d'insectes porteurs du parasite. Le second stade correspond au passage du parasite dans le foie et à l'infection des cellules hépatiques dans lequelles les parasites se développent pour former les schizontes hépatiques qui libèrent par éclatement les mérozoïtes hépatiques. Le troisième stade est caractérisé par l'infection des érythrocytes sanguins par les formes asexuées (mérozoïtes) du parasite ; ce stade érythrocytaire de développement du parasite correspond à la phase pathogène de la maladie. Le quatrième stade correspond à la formation des formes sexuées (ou gamétocytes) qui deviendront les gamètes extra-cellulaires chez le moustique.

On sait que de nombreuses études ont été entreprises pour isoler à partir des souches de parasites infectantes pour un hôte humain des fractions polypeptidiques, d'une part pour assurer le diagnostic in vitro du paludisme par détection des anticorps correspondants, et, d'autre part, pour tenter de vacciner contre le paludisme,

Par exemple, des banques de cADNs clonés dérivés des sporozoïtes de Plasmodium falciparum ont été établies par ENEA et coll (1984) Science, vol. 225, 628-630. Il a été reconnu que ces banques comportaient des clones susceptibles d'exprimer des polypeptides immunogéniques contenant des unités répétitives de 4 acides aminés spécifiques de l'antigène circumsporozoïtaire (de P. falciparum).

Toutefois, peu de travaux ont été effectués sur les formes hépatiques des parasites responsables du paludisme. La morphologie des formes hépatiques a été décrite pour la première fois en 1948 à partir de biopsies de volontaires humains infectés (Trans. Roy. Soc. Trop. Med. Hyg., 41, 785 (1948)). Un antigène spécifique du stade hépatique de P. falciparum a pu être décrit dans le foie de singes d'Amérique du Sud insensibles aux formes sanguines du parasite, mais chez lesquels les formes hépatiques peuvent se développer (Am. J. Trop. Med. Hyg., 33, (3) 336-341 (1984)).

La détection de la localisation du ou des antigène(s) spécifique(s) du foie (ci-après désigné par LSA pour "Liver Specific Antigen") a été réalisée par immunofluorescence tout au long des étapes de maturation du schizonte. Il est localisé à la périphérie du parasite de taille 5 à 40 microns ; par la suite il est distribué entre les cytomères ou paquets de mérozoïtes, lorsque les schizontes atteignent entre 50 et 100 microns. Il se distingue des antigènes de surface des sporozoïtes et des antigènes partagés par les schizontes du sang et du foie qui donnent une image d'immunofluorescence interne au parasite.

Bien qu'il soit désormais possible de cultiver des formes hépatiques de P.falciparum dans des hépatocytes humains (Science, 227, 440 (1985)), le faible taux d'obtention de formes matures du parasite par les méthodes de culture in vitro et in vivo ne permet pas l'analyse biochimique de l'antigène produit au stade hépatique.

Il a également été observé que les individus atteints de paludisme possèdent un taux d'anticorps dirigés contre le LSA très élevé. Le LSA semble être un immunogène très puissant, parmi les plus puissants de tous les antigènes synthétisés aux différents stades de développement du parasite. Par contre, le taux d'anticorps dirigés contre les formes des stades sanguins du parasite est parfois très faible, et de ce fait le diagnostic réalisé à partir d'antigènes spécifiques des stades sanguins peut parfois être faussement négatif.

Un des buts de la présente invention est précisément de permettre le diagnostic in vitro de l'infection d'un individu par P. falciparum dans des conditions plus sensibles que ne le permettent les méthodes actuelles.

L'invention a également pour objet de nouvelles compositions pour la vaccination chez l'homme contre le paludisme provoqué par P. falciparum.

L'invention concerne plus particulièrement des molécules, ou compositions peptidiques ou polypeptidiques, caractérisées par la présence dans leur structure d'une ou de plusieurs séquences peptidiques porteuses d'un ou plusieurs épitopes caractéristiques de la protéine résultant de l'activité infectieuse de P. falciparum dans les cellules hépatiques, notamment de l'épitope B.

Une composition polypeptidique selon l'invention est essentiellement caractérisée par la présence d'une séquence de 17 acides aminés de formule : 
Leu-Ala-Lys-Glu-Lys-Leu-Gln-X-Gln-Gln-Ser-Asp-Leu-Glu-
Gln-Glu-Arg
dans laquelle :
X est Glu ou Gly
et dans laquelle "Leu" est la leucine, "Ser" est la sérine, "Lys" est la lysine, "Glu" est l'acide glutamique, "Gln" est la glutamine, "Asp" est l'acide aspartique, et "Arg" est l'arginine. Lorsque X est "Glu", on désignera dans la suite de ce texte le peptide correspondant par "peptide I", et lorsque X est "Gly" par "peptide II".

L'invention concerne en premier lieu des peptides monomères synthétiques comprenant une séquence peptidique unique de 17 acides aminés répondant respectivement à la formule sus-indiquée, et dont les acides aminés terminaux possèdent des extrémités respectivement amine et carboxylique libres, ou des oligomères contenant notamment des multiples de l'une quelconque des deux susdites séquences peptidiques I et II de 17 acides aminés.

Il va de soi que les fonctions réactives libres que sont susceptibles de posséder certains acides aminés entrant dans la constitution des molécules selon l'invention, notamment les groupes carboxyles libres portés par les groupes Glu ou par l'acide aminé C-terminal, d'une part, et/ou les groupes libres portés par l'acide aminé N-terminal ou par des acides aminés intérieurs à la chaîne peptidique, par exemple Lys, d'autre part peuvent être modifiées, dès lors que cette modification n'entraîne pas une modification des propriétés antigéniques, le cas échéant immunogèniques, de l'ensemble de la molécule. Les molécules ainsi modifiées entrent naturellement dans le cadre de la protection donnée à l'invention par les revendications. Ces fonctions carboxyles sont éventuellement acylées ou estérifiées.

D'autres modifications entrent également dans le cadre de l'invention. En particulier, fonctions amine ou ester, ou les deux à la fois, des acides aminés terminaux peuvent être engagées elles-mêmes dans des liaisons avec d'autres acides aminés. Par exemple l'acide N-terminal peut être lié à une séquence comprenant de 1 à plusieurs acides amines correspondant à une partie de la région C-terminale d'un autre peptide conforme à la définition qui en a été donnée plus haut, ou vice-versa.

Il va de soi également que toute séquence peptidique issue de la modification, par substitution et/ou par addition et/ou suppression d'un ou plusieurs acides aminés, de l'une ou l'autre des deux séquences peptidiques I et II de 17 acides aminés, entre dans le cadre de la protection donnée à l'invention par les revendications, dès lors que cette modification n'altère pas les propriétés antigéniques ou immunogéniques des polypeptides I et II, notamment lorsque ces propriétés immunogéniques ont été renforcées de façon adéquate, par exemple par association de ces polypeptides I ou II avec un adjuvant immunologique approprié ( par exemple un muramylpeptide) ou par couplage avec une molécule porteuse de poids moléculaire plus élevé (par exemple une sérum-albumine ou une poly-lysine) ou une toxine du type tétanique ou un autre antigène de P.falciparum.

L'invention concerne plus généralement toute molécule caractérisée par la présence dans sa structure d'une ou plusieurs séquences peptidiques présentant des réactions immunologiques croisées avec les deux séquences peptidiques répondant à la formule précédente vis-à-vis des anticorps inductibles par ces dernières in vivo.

L'invention concerne aussi tout peptide, dont la structure découle de ceux qui ont été précédemment indiqués, par une permutation circulaire, d'un ou plusieurs aminoacides. En particulier elle concerne aussi des peptides de formule : 
Ala-Lys-Glu-Lys-Leu-Gln-X-Gln-Gln-Ser-Asp-Leu-Glu-Gln-
Glu-Arg-Leu

Lys-Glu-Lys-Leu-Gln-X-Gln-Gln-Ser-Asp-Leu-Glu-Gln-Glu-
Arg-Leu-Ala

Glu-Lys-Leu-Gln-X-Gln-Gln-Ser-Asp-Leu-Glu-Gln-Glu-Arg-
Leu-Ala-Lys

Lys-Leu-Gln-X-Gln-Gln-Ser-Asp-Leu-Glu-Gln-Glu-Arg-Leu-
Ala-Lys-Glu

Leu-Gln-X-Gln-Gln-Ser-Asp-Leu-Glu-Gln-Glu-Arg-Leu-Ala-
Lys-Glu-Lys

Gln-X-Gln-Gln-Ser-Asp-Leu-Glu-Gln-Glu-Arg-Leu-Ala-Lys-
Glu-Lys-Leu

X-Gln-Gln-Ser-Asp-Leu-Glu-Gln-Glu-Arg-Leu-Ala-Lys-Glu-
Lys-Leu-Gln

Gln-Gln-Ser-Asp-Leu-Glu-Gln-Glu-Arg-Leu-Ala-Lys-Glu-Lys-
Leu-Gln-X-

Gln-Ser-Asp-Leu-Glu-Gln-Glu-Arg-Leu-Ala-Lys-Glu-Lys-Leu-
Gln-X-Gln

Ser-Asp-Leu-Glu-Gln-Glu-Arg-Leu-Ala-Lys-Glu-Lys-Leu-Gln-
X-Gln-Gln-

Asp-Leu-Glu-Gln-Glu-Arg-Leu-Ala-Lys-Glu-Lys-Leu-Gln-X-
Gln-Gln-Ser-
 
Leu-Glu-Gln-Glu-Arg-Leu-ala-Lys-Glu-Lys-Leu-Gln-X-Gln-
Gln-Ser-Asp

Glu-Gln-Glu-Arg-Leu-Ala-Lys-Glu-Lys-Leu-Gln-X-Gln-Gln-
Ser-Asp-Leu

Gln-Glu-Arg-Leu-Ala-Lys-Glu-Lys-Leu-Gln-X-Gln-Gln-Ser-
Asp-Leu-Glu

Glu-Arg-Leu-Ala-Lys-Glu-Lys-Leu-Gln-X-Gln-Gln-Ser-Asp-
Leu-Glu-Gln

Arg-Leu-Ala-Lys-Glu-Lys-Leu-Gln-X-Gln-Gln-Ser-Asp-Leu-
Glu-Gln-Leu
dans lesquels X a la signification sus-indiquée.

Comme dans le cas du premier peptide sus-défini, les différents peptides qui viennent d'être nommés peuvent être modifiés sans pour autant sortir du cadre de l'invention, dès lors que ces modifications de structure n'entraîneraient pas des transformations profondes de leurs propriétés antigéniques.

Las peptides selon l'invention peuvent être préparés par les techniques classiques, dans le domaine de la synthèse des peptides. Cette synthèse peut être réalisée en solution homogène ou en phase solide.

Par exemple, on aura recours à la technique de synthèse en solution homogène décrit par HOUBENWEYL dans l'ouvrage intitulé "Meuthods der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide. Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De péférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acide aminés constituant la chaîne peptidique et on détache le peptide de la résine par exemple à l'aide d'acide fluorydrique.

L'invention concerne également les oligomères hydrosolubles des peptides monomères sus-indiqués. L'oligomérisation peut provoquer un accroîssement de l'immunogénicité des peptides monomères selon l'invention. Sans qu'une telle indication chiffrée puisse être considérée comme limitative, on mentionnera néanmoins que ces oligomères peuvent, par exemple, contenir de 2 à 10 unités monomères.

Les unités monomères entrant dans cet oligomère sont soit toutes constituées par le polypeptide de séquence I ou par le polypeptide de séquence II, soit par l'un et l'autre de ces polypeptides.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

On peut également pour la production de molécules comportant un ou plusieurs motifs de 17 acides aminés tels que définis ci-dessus, avoir recours à des techniques du génie génétique mettant en oeuvre des micro-organismes transformés par un acide nucléique déterminé comprenant des séquences nucléotidiques appropriées correspondantes.

A ce titre, l'invention concerne également des acides nucléiques contenant une ou plusieurs de ces séquences répétitives comprenant chacune 17 triplets du genre sus-indiqué.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit :

A titre d'exemple de molécules porteuses ou supports macromoléculaires entrant dans la constitution des conjugués selon l'invention, on mentionnera des protéines naturelles, telles que l'anatoxine tétanique, l'ovalbulmine, des sérums albumines, des hémocyamines, etc...

A titre de supports macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20 000.

Pour synthétiser les conjugués selon l'invention, on peut avoir recours à des procédés connus en soi, tels que celui décrit par FRANTZ et ROBERTSON dans Infect. and Immunity, 33, 193-198 (1981), ou celui décrit dans Applied and Environmental Microbiology, (octobre 1981), vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N-éthyl-N'(3-diméthylamino-propyl) carbodiimide, HCl], diisocyanates, bis-diazobenzidine, di- et trichloro-s-triazines, bromures de cyanogène, ainsi que les agents de couplage mentionnés dans Scand. J. Immunol., (1978), vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives de molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminopropyl)-carbodiimide, le N-hydroxybenzotriazole, etc... On peut encore avoir recours à la glutaraldéhyde, notamment lorsqu'il s'agit de relier entre eux des groupes aminés respectivement portés par le peptide et la molécule support.

Un groupe de molécules préférées selon l'invention est constitué de celles possédant une conformation en hélice α, cette dernière renforçant les propriétés antigéniques, et immunogéniques desdites molécules. De telles molécules possédant une conformation en hélice α ont été mise en évidence par dichroisme circulaire dans le trifluoroethanol, ou en solution aqueuse.

Les molécules selon l'invention possèdent des propriétés antigéniques caractéristiques de l'antigène LSA sépcifique du stade hépatique du développement de P. falciparum.

En effet, comme il le sera plus particulièrement décrit à l'aide d'exemples de molécules selon l'invention dans la description détaillée qui suit, les molécules selon l'invention réagissent spécifiquement avec les anticorps dirigés contre l'antigène LSA produit par P. falciparum, mais pas avec les anticorps dirigés contre d'autres antigènes produits par P. falciparum ou contre des antigènes produits par d'autres espèces de Plasmodium.

Ces molécules selon l'invention reconnaissent donc spécifiquement les anticorps produits par le système immunitaire d'un individu infecté par P. falciparum sous l'effet de l'antigène LSA dont le caractère fortement immunogène a été précédemment mentionné.

Ainsi la possibilité de production en grande quantité des molécules selon l'invention ainsi que leurs propriétés de reconnaissance spécifique des anticorps les plus activement produits lors de l'infection d'un individu par P. falciparum, font desdites molécules des réactifs de choix pour le diagnostic in vitro du paludisme chez un individu infecté par P. falciparum.

L'invention concerne donc un procédé de détection in vitro d'anticorps corrélables au paludisme issu de l'infection d'un individu par P. falciparum dans un tissu ou fluide biologique susceptible de les contenir, ce procédé comprenant la mise en contact de ce tissu ou fluide biologique avec une molécule selon l'invention dans des conditions permettant une réaction immunologique in vitro entre lesdites molécules et les anticorps éventuellement présents dans le tissu ou fluide biologique, et la détection in vitro du complexe antigène-anticorps éventuellement formé.

De préférence, le milieu biologique est constitué par un sérum humain.

Toute procédure classique peut être mise en oeuvre pour réaliser une telle détection.

A titre d'exemple une méthode préférée met en jeu des processus immunoenzymatiques selon la technique ELISA, ou immunofluorescents, ou radioimmunologiques (RIA) ou équivalent.

Ainsi l'invention concerne également toute molécule selon l'invention marquée à l'aide d'un marqueur adéquat du type enzymatique, fluorescent, radioactif, etc...

De telles méthodes comprennent par exemple les étapes suivantes :
- dépôt de quantités déterminées d'une composition polypeptidique selon l'invention dans les puits d'une microplaque de titration,
- introduction dans lesdits puits de dilutions croissantes du sérum devant être diagnostiqué,
- incubation de la microplaque,
- rinçages répétés de la microplaque,
- introduction dans les puits de la microplaque d'anticorps marqués contre des immunoglobulines du sang, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée,
- détection, en comparaison avec un témoin de contrôle, de la quantité de substrat hydrolysé.

L'invention concerne également des coffrets ou kits pour le diagnostic in vitro du paludisme provoqué par P. falciparum qui comprennent :
- une composition polypeptidique selon l'invention,
- les réactifs pour la constitution du milieu propice à la réalisation de la réaction immunologique,
- les réactifs permettant la détection du complexe antigènes-anticorps produit par la réaction immunologique. De tels réactifs peuvent également porter un marqueur, ou être susceptibles d'être reconnus à leur tour par un réactif marqué. Plus particulièrement dans le cas où la composition polypeptidique sus-mentionnée n'est pas marquée.
- un tissu fluide biologique de référence dépourvu d'anticorps reconnus par la composition polypeptidique sus-mentionnée,
L'invention concerne les anticorps eux-mêmes formés contre les polypeptides de l'invention.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisé contre l'un des polypeptides purifiés de l'invention, d'une part et des cellules d'une lignée de cellule myélome approprié d'autre part, et d'être sélectionné, par sa capacité à produire des anticorps monoclonaux reconnaissant le polypeptide initialement mis en oeuvre pour l'immunisation des animaux, c'est-à-dire plus particulièrement le polypeptide I ou le polypeptide II.

L'invention ouvre enfin la voie à la mise au point de nouveaux principes vaccinants contre le paludisme issu de l'infection d'un individu par P. falciparum.

L'invention concerne également les compositions préparées sous forme de vaccins contenant soit le peptide selon l'invention, soit un oligomère de ce peptide, soit encore un conjugué de ce peptide ou oligomère avec une molécule porteuse, en association avec un véhicule pharmaceutiquement acceptable approprié et, le cas échéant, avec d'autres principes actifs vaccinants contre le paludisme.

Des compositions pharmaceutiques avantageuses sont constituées par des solutions, suspensions ou liposomes injectables contenant une dose efficace d'au moins un produit selon l'invention. De préférence, ces solutions, suspensions ou liposomes sont réalisés dans une phase aqueuse stérilisée isotonique, de préférence saline ou glucosée.

L'invention concerne plus particulièrement de tel les suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des produits selon l'invention se trouve associé à des excipients pharmaceutiquement acceptables, solides ou liquides, adaptés à la constitution de formes orales, oculaires ou nasales, ou avec des excipients adaptés à la constitution des formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaires ou nasales.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthylcellulose, les hydroxydes et phosphates d'aluminium ou tous autres adjuvants de ce type, bien connus de l'homme de l'art. Enfin elles contiennent si besoin un adjuvant immunologique, notamment du type muramylpeptide.

L'invention ne se limite évidemment pas aux modes de réalisation décrits ci-dessus à titre d'exemples et l'homme de l'art peut y apporter des modifications sans pour autant sortir du cadre des revendications ci-après ; notamment certains des acides aminés intervenant dans la séquence des peptides selon l'invention peuvent être remplacés par des acides aminés isofonctionnels ou isostériques ; par exemple, une ou plusieurs des substitutions suivantes peuvent être envisagées :
- Glu est substitué par Asp ou Gln,
- Leu est remplacé par Ala, etc...

Il est naturellement bien entendu que les peptides qui résultent de telles substitutions consistent en des équivalents des peptides plus particulièrement revendiquées, dès lors qu'eux-mêmes ou des oligomères ou conjugués formés à partir de ces peptides présentent des propriétés immunogéniques semblables.

L'invention concerne également encore plus particulièrement les "protéines chimères" qui peuvent être obtenues par les techniques du génie génétique, ces protéines chimères pouvant contenir une ou plusieurs séquences peptidiques, comportant respectivement les 17 acides aminés des séquences de l'invention, et incorporées ou rattachées à un fragment peptidique autre que la β-galactosidase. Ce dernier fragment peptidique a de préférence un poids moléculaire suffisant pour renforcer l'immunogénicité des séquences peptidiques selon l'invention et n'interfère pas du point de vue immunologique avec la manifestation de l'immunogénicité recherchée.

Des caractéristiques supplémentaires de l'invention apparaîtront encore, au cours de la description qui suit, des conditions dans lesquelles un polypeptide contenant une pluralité de séquences de 17 acides aminés selon l'invention a été obtenu.

Il sera fait référence dans ce qui suit aux dessins dans lesquel
- la figure 1 fournit la séquence nucléotidique d'un des acides nucléiques recombinants étudiés (clone DG 307) lequel contient lui-même une séquence spécifique codant pour un polypeptide caractéristique des formes hépatiques de P. falciparum ;
- la figure 2 fournit la séquence en acides aminés codée pour la susdite séquence nucléotidique spécifique.

Des sérums provenant d'individus européens vivant dans des zones endémiques et suivant une prophylaxie continue avec des médicaments dirigés contre les schizontes des stades sanguins ont été sélectionnés et testés en utilisant des antigènes du stade des sporozoïtes (antigènes CS), du stade hépatique (antigène LSA), et des stades sanguins. La plupart de ces sérums réagissent avec les antigènes de tous les stades probablement car la prophylaxie a été interrompue. Trois sérums prélevés à partir d'individus ayant résidé en Afrique tropicale rurale et ayant ingéré 100 µg de chloroquine par jour sans interruption pendant 23 à 26 ans, ne réagissent pas avec les antigènes des stades sanguins suivant le test d'immunofluorescence (IFA). Ces trois sérums possédant toutefois des titres élevés en anticorps dirigés contre les sporozoïtes et les protéines LSA (dilution IFA 1/3200 et 1/6400 respectivement).

Un des trois sérums précédents , de spécificité réduite, a été utilisé pour cribler une banque d'ADN génomique construite dans le bactériophage λ gt 11 de la manière suivante :

### 1) CONSTRUCTION DE LA BANQUE D'ADN GENOMIQUE DE Plasmodium falciparum.

L'ADN génomique du clone 96 de la souche thailandaise Tak9 de P.falciparum (Science, 212, 1.37-1.38 (1981) a été isolé par les techniques classiques.

Des échantillons de 18 µg d'ADN de P.falciparum ont été incubés à 15°C dans un tampon 50 mM Tris HCl pH 7.5, 1 mM MnCl₂, 20 µg/ml de sérum albumine bovine, avec des quantités respectives d'ADNase I (Boehringer Mannheim) de 5 pg pendant 5 minutes ou bien de 3.5 pg pendant 5 ou 10 minutes. Après addition de 5 mM EDTA (Ethylènediamine tetracétique acide), les échantillons d'ADN sont réunis et purifiés par extraction au mélange phénol/chloroforme/alcool isoamylique (25 V/24 V/1 V) puis par l'éther. L'ADN est concentré par précipitation à l'éthanol à -20°C en présence de 2.5 M d'acétate d'ammonium.

45 µg d'ADN ainsi traité ont été méthylés par 180 U d'Eco R1 méthylase (Biolabs) dans les conditions recommandées par le fournisseur, avec l'addition supplémentaire de 5 mM DTT (dithiothreitol), pendant 15 minutes à 37° C. Après purification de l'ADN comme ci-dessus, 10 µg d'ADN ont été incubés avec 40 mM Tris HCl pH 8.0, 10 mM sulfate d'ammonium, 10 mM 2-mercaptoethanol, 0.5 m M EDTA, 0.05 mM NAD (nicotinamide adénine dinucléotide) 0.1 mM dXTP (comprenant les 4 desoxynucleotides triphosphates dATP, dCTP, dGTP et dTTP), en présence de 10 U T4 ADN polymérase (PL Biochemicals) et de 10 U de E. coli ADN ligase (Biolabs). L'ADN a été purifié et concentré comme ci-dessus.

8 µg d'ADN ont alors été ligaturés avec 0.4 µg d'un adaptateur ou "linker" Eco R1 (adaptateurs phosphorylés Eco R1 commercialisés par Biolabs) par 4 U T4 ADN ligase (Biotec) dans le tampon 50 mM Tris HCl pH 8.0, 10 mM MgCl₂, 20 mM DTT, 1 mM ATP, 50 µg/ml sérum albumine bovine.

Après incubation à 4° C pendant 5 heures, on ajoute 2 U T4 ADN ligase et la réaction est poursuivie à 4° C pendant 16 heures. Le tube est soumis à plusieurs cycles de congélation à -80° C / décongélation pour arrêter la réaction. L'ADN est ensuite dilué et le tampon d'incubation ajusté de façon à obtenir les conditions recommandées par le fournisseur pour l'utilisation de l'enzyme Eco R1. 100 U d'enzyme Eco R1 (Promega Biotec) sont ajoutées et incubées pendant 3 heures à 37°C. La réaction est arrêtée par un chauffage de 10 minutes à 60° C, et l'ADN est purifié et concentré comme ci-dessus.

L'ADN est resuspendu dans 100 µl de tampon 50 mM Tris HCl pH 8.0, 1 mM EDTA, et déposé sur un gradient 5-20 % de saccharose préparé en 25 mM acétate de sodium, 10 mM EDTA et centrifugé dans le rotor Beckman SW 50.1 à 45,000 tours par minute pendant 150 minutes. Les fractions sont analysées sur gel d'agarose et celles qui contiennent les fragments d'ADN de tailles comprises entre environ 300 pb. et 2 500 pb. sont rassemblées, dialysées contre le tampon 50 mM Tris HCl pH 8.0, 1 mM EDTA à 4°C. L'ADN est concentré par précipitation à l'éthanol. Environ 400 ng de cet ADN ont été ligaturés à 1 µg d'ADN du vecteur gt11 (Proc. Natl. Acad. Sci., USA, 80, 1194-1198 (1983)) coupé par Eco R1 et déphosphorylé (Protoclone de Promega Biotec), dans un volume de 10µl, (en tampon 50 mM Tris HCl pH 8.0, 10 mM MgCl₂, 20 mM DTT, 1 mM ATP, 50 µg/ml sérum albumine bovine) par 1 U T4 ADN ligase (Biotec).

Les produits de ligature ont été encapsidés in vitro dans les extraits d'E. coli préparés à partir des souches bactériennes construites par B. Hohn (Methods Enzymol. 68, 299), selon la technique décrite par Maniatis et coll. (Molecular cloning, a laboratory manual, p. 264, Cold Spring Harbor Laboratory (1982)). Environ 7 millions de bactériophages recombinants ont été obtenus.

### 2) CRIBLAGE IMMUNOLOGIQUE DE LA BANQUE

Les bactériophages recombinants ont été étalés sur un milieu de culture contenant la bactérie indicatrice Y 1090, à une densité de 50,000 plages par boite de Pétri de 90 mm, et incubés à 42°C pendant 3 heures. Un filtre de nitrocellulose (Schleicher & Schuell, BA 85) sature par 0,01 M IPTG isopropyl-β-thiogalactopyranoside (Sigma) est déposé sur les boites, qui sont incubées à 37°C pendant 3 heures. Au terme de ces incubations, les filtres de nitrocellulose sont prélevés, et les boites de Pétri conservées à 4° C.

Les filtres de nitrocellulose sont placés dans un bain de tampon TL : 50 mM tris HCl pH 8.0, 150 mM NaCl, 5 % lait écrémé, 0.05 % Tween 20 (Sigma). Les filtres sont incubés 15 heures à 4° C en tampon TL, puis 2 fois 15 minutes à 20° C. Ils sont alors incubés pendant une heure avec un pool d'antisérums humains immuns dirigés contre les antigènes de tous les stades de développement de P. falciparum, traité au préalable pour le dépléter en anticorps anti-E.coli selon la technique décrite par Ozaki et coll. (J. Immunol. Methods, 89, 213-219, 1986). Le pool d'antisérums humains a été utilisé à la dilution 1/200, en tampon TL. L'incubation a été faite à 20°C pendant 1 heure. Les filtres ont été lavés 4 fois avec le tampon TL, puis incubés avec des anticorps anti-immunoglobulines humaines conjugués à la peroxydase de raifort (Biosys) et iodinés à l'iode ¹²⁵I, pendant 1 heure à 20° C. Après plusieurs lavages en tampon TL, puis en tampon 50 mM Tris HCl pH 8.0, 150 mM NaCl, l'activité enzymatique de la peroxydase est révélée (Ozaki et coll, précédemment cité), les filtres sont séchés à l'air libre et autoradiographiés sur film Kodak Royal X-OMat AR, avec un écran amplificateur.

Une collection d'environ 1 200 clones de bactériophages recombinants a été constituée en prélevant les plages de lyse correspondant aux signaux positifs. Ces clones ont été ensuite soumis à un second cycle de criblage immunologique, en employant cette fois un des trois sérums humains précédemment décrits (p.16 et 17) et présentant pas ou peu d'anticorps dirigés contre les formes érythrocytaires de P. falciparum et un titre élevé contre les formes hépatiques du parasite. Ce criblage immunologique a été effectué selon le protocole décrit ci-dessus. Ce sérum réagit avec seulement 15 % des clones producteurs d'un antigène spécifique de P.falciparum (60 sur 400 testés) et parmi les clones les plus actifs, 22 ont été sélectionnés et caractérisés comme suit.

Les anticorps humains qui réagissent avec les déterminants antigéniques exprimés par les clones recombinants ont été purifiés par affinité sur les protéines recombinantes, selon la technique décrite par Ozaki et coll. (précédemment cité). Ces anticorps spécifiques ont été incubés avec des préparations de parasites à différents stades de développement (sporozoite, stade hépatique ou stades érythrocytaires), et la réaction a été étudiée par immunofluorescence indirecte. Les clones recombinants sur lesquels sont retenus par affinité des anticorps spécifiques du stade hépatique, et donc qui expriment des déterminants propres à ce stade ont été étudiés : il s'agit des clones DG 307, DG 199 et DG 145. Ces anticorps spécifiques de ces trois clones réagissent spécifiquement avec les schizontes hépatiques, tels que l'on peut les obtenir après infection d'hépatocytes humains ou de singes par des sporozoïtes de P.falciparum ; la localisation de la fluorescence a été déterminée comme étant identique à celle considérée caractéristique de LSA.

La spécificité d'espèce et de stade des 3 clones DG 145, DG 199, et DG 307 a été testée de la manière suivante. Premièrement, il a été déterminé que les mêmes anticorps purifiés par affinité et qui réagissent par IFA (ou encore qui sont IFA positifs) avec LSA, ne réagissent pas avec des préparations de sporozoïtes sèches ou humides, ni avec les antigènes des stades sanguins, qu'ils soient testés par IFA avec des parasites fixés à l'acétone, ou par immunotransfert (immunoblotting) en utilisant des protéines de tous les stades extraites au SDS. Les anticorps purifiés par affinité ne réagissent pas avec les antigènes de stade hépatique de P. yoelii, ni avec les schizontes hépatiques de P. vivax préparés à partir de singes Saimiri sciureus.

Deuxièmement, les protéines recombinantes de DG 145, DG 199 et DG 307 ne réagissent pas avec les sérums provenant de deux patients atteints de malaria (paludisme causé par P.falciparum) par transfusion accidentelle et qui, par définition n'ont donc pas d'anticorps contre les antigènes spécifiques des stades précédents (sporozoïtes et antigènes du stade hépatique). Ces protéines ne réagissent pas avec deux anticorps monoclonaux reconnaissant le tétrapeptide CS, avec les sérums de souris immunisées avec les antigènes CS recombinants R32t et 32 (Science, 228, 958 (1985)). De plus, les protéines recombinantes n'ont pas réagi avec des antisérums humains dirigés contre P. vivax (bien que les sérums furent positifs avec les schizontes hépatiques de P. vivax), P. ovale et P. cynomolgi (Ann. Soc. Belg. Med. Trop., 60, 348 (1980)) quand elles sont testées par la technique de taches d'immunotransfert (immunodot blots), alors qu'elles sont positives avec tous les sérums humains anti-P. falciparum testés.

La stabilité à la chaleur, connue comme étant une caractéristique du LSA mature (Ann. J. Trop. Med. Hyg., 33 (3) 336-341 (1984)) a également été démontrée pour la protéine de fusion produite par le clone DG 307. Elle demeure antigéniquement active après un traitement de 15 minutes à 100°C . Pour cette raison le clone DG 307 a été analysé plus en détail.

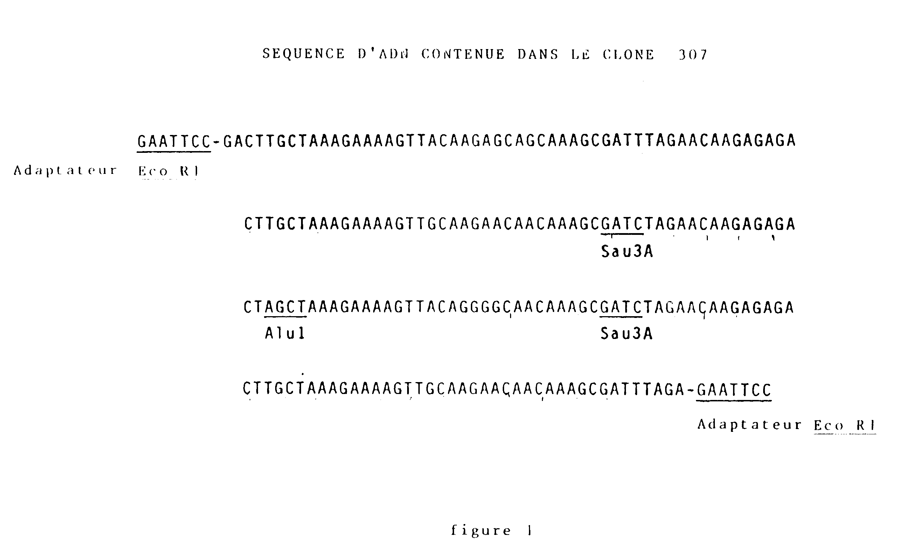

L'insérat de 196 paires de bases de P. falciparum a été purifié et recloné à l'intérieur du plasmide pUC13 et du bactériophage M13 mp 8 (Nucleic Acids Research, 9, 309-321 (1981)) . La séquence d'ADN et l'organisation génomique du gène LSA ont alors été déterminées. La figure 1 montre que le clone DG 307 contient un fragment d'ADN composé entièrement de répétition d'un motif de 51 paires de bases.

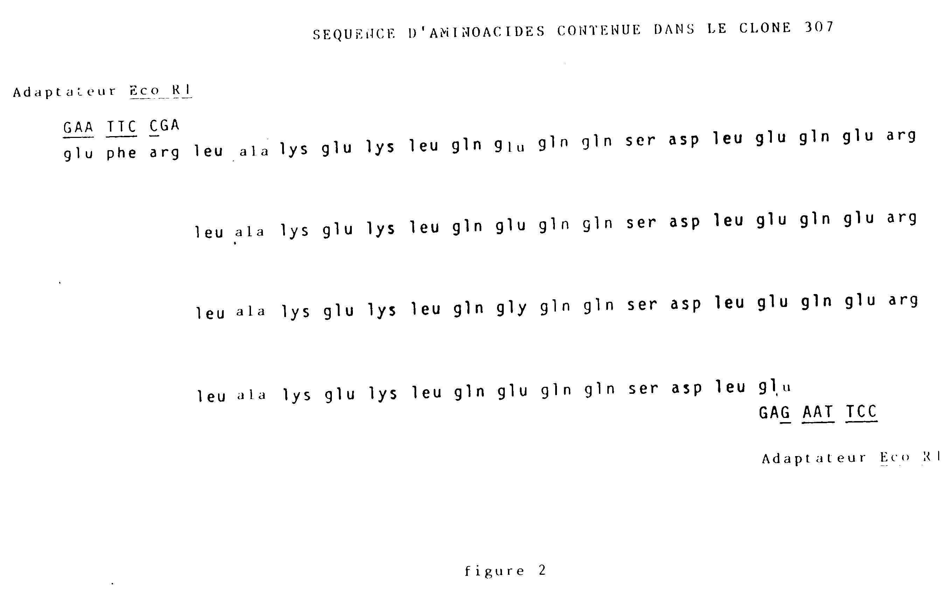

Seulement une phase de lecture est en phase avec le gène lac Z de la β-galactosidase, une caractéristique attendue puisque le clone produit une protéine de fusion qui porte les épitopes reconnues par le sérum humain. La séquence en acides aminés correspondant à ce fragment est représentée à la figure 2. Elle est composée d'une répétition de 17 acides aminés riche en glutamine, acide glutamique, et leucine. L'analyse informatique de cette séquence indique qu'elle a une grande probalité de posséder une structure en hélice α sans pli β. De plus, aucune homologie entre les séquences d'ADN connues à l'heure actuelle et la séquence d'ADN codant pour ladite protéine n'a été détectée par analyse des banques de données Los Alamos et NBRF. Ceci est en accord avec la spécificité du stade hépatique de la protéine LSA.

Des analyses par transfert selon la méthode de Southern utilisant le fragment de 196 paires de bases cloné dans pUC 13 indiquent que ce fragment est un dérivé d'un gène unique puisqu'il s'hybride à des fragments uniques Eco RI (6,5 kb) et Dra I (4,5 kb). Le gène semble être polymorphe, car des fragments Rsa I de différentes tailles ont été observés lorsque de l'ADN provenant d'une autre souche a été utilisé. Le gène a été trouvé dans toutes les souches de P. falciparum examinées jusqu'à maintenant, localisé sur un des plus grands chromosomes. Les études d'hybridation d'ADN indiquent que les trois clones DG 307, 145, et 199 sont des fragments séparés dérivés du même gène.

Le type de répétition codée par DG 307 semble être conservé et implique que le ou les épitopes correspondant pourraient être présents chez toute les souches de P. falciparum. Ceci est en accord avec l'observation du fait que le gène qui est dérivé de la souche Thai Tak9.96 a été détecté en utilisant du sérum humain d'Afrique ou de personnes immunisées par les souches africaines.

Comme les épitopes répétitifs apparaissent être une caractéristique générale des antigènes de P. falciparum (Nature, 306, 751-756 (1983) ; Nature, 311, 382-385 (1984) ; Science, 225, 593-599 (1984) ; Science, 227, 1595-1597 (1985) ; Cell, 40, 775-783 (1985)), il n'est pas surprenant que le LSA qui est hautement immunogène chez l'homme possède également des structures répétitives. La forte réaction du clone DG 307 avec les antisérums humains indique que l'épitope naturel est défini par moins de 4 répétitions.

La relation entre le, ou les, épitope(s) exprimé(s) par la protéine recombinante de DG 307, et les épitopes présents dans la protéine mature du stade hépatique, a été étudiée de la manière suivante : 10 sérums humains provenant de Côte d'Ivoire , Gabon et Zaïre, possédant des titres élevés en anticorps dirigés contre ladite protéine mature ont été ajustés à des dilutions présentant une nette réaction positive IFA avec les antigènes du stade hépatique. Par la suite, ces sérums ont été incubés soit en présence de l'extrait protéique du clone DG 307 induit par l'IPTG, soit avec des protéines provenant de deux autres clones exprimant des déterminants antigéniques des stades sanguins. Les concentrations des antigènes ont été ajustées à un niveau de réactivité similaire au précédent en testant plusieurs dilutions par immunodot blots et en révélant l'anticorps lié en utilisant un pool de sérums hyperimmuns. Après incubation pendant 2 heures à température ambiante, la spécificité pour les schizontes hépatiques des différentes solutions a été testée par IFA. Les résultats indiquent que les anticorps préincubés avec l'extrait de DG 307 n'ont plus aucune réactivité IFA, alors que ceux incubés avec les deux autres extraits n'ont pas vu leur activité contre l'antigène hépatique diminuér.

Il semble donc que le DG 307 code pour l'épitope principal du LSA puisque dans ces expériences d'absorption, la réactivité IFA à la protéine mature a été abolie pour 9 des 10 sérums humains testés, alors qu'aucune diminution de l'activité IFA, n'a été observée après pré-incubation de ces mêmes 10 sérums en présence de protéines recombinantes différentes de celles de DG 307.

L'invention concerne encore les acides nucléiques recombinants contenant l'une au moins des séquences polypeptidiques I ou II, ou les deux à la fois, ainsi que les micro-organismes, notamment les bactéries E.coli tranformées par ces acides nucléiques recombinants et capables d'exprimer lesdits polypeptides.

L'invention concerne ces séquences d'acides nucléiques ou des séquences équivalentes qui peuvent être synthétisées et qui codent pour les mêmes acides aminés.

Il apparaîtra immédiatement à l'homme de métier que dans ces séquences, certains des nucléotides peuvent être remplacés par d'autres en raison de la dégénéréscence du code génétique sans que pour autant les peptides codés ne soient modifiés. Toutes ces séquences nucléotidiques, ainsi que celles qui codent pour des polypeptides qui diffèrent des précédents par un ou plusieurs acides aminés sans que leur activité immunogéique propre ne soit modifiée de façon semblable, font partie de l'invention. Il en va naturellement de même des séquences nucléotidiques qui peuvent être reconstituées et qui sont capables de coder pour des oligomères tels qu'ils ont été définis plus haut. Les motifs monomères sont liés directement bout à bout ou par l'intermediaire de séquences peptidiques sans effet sur les propriétés immunogéniques des oligomères ainsi formés.

Enfin, l'invention concerne les vecteurs modifiés par ces micro-organismes, ces vecteurs étant naturellement pourvus de régulation et de terminaison précédant et suivant les séquences nucléiques sus-indiquées, qui permettront l'expression de ces dernières dans des organismes cellulaires compétents.

Parmi les séquences nucléotidiques qui codent pour les peptides caractéristiques qui ont été définis ci-dessus, on mentionnera celles qui sont caractérisées par les séquences de triplets qui suivent, ces séquences correspondant en particulier pour les trois premières au peptide I et pour la dernière au peptide II dont les formules ont été précédemment indiquées (étant entendu que les nucléotides qui sont remplacés par des points dans les séquences ci-après sont identiques à ceux de la première séquence à l'aplomb duquel ils se trouvent).

(1)CTT GCT AAA GAA AAG TTA CAA GAG CAG CAA AGC GAT TTA GAA CAA GAG AGA
(2)... ... ... ... ... ..G ... ..A ..A ... ... ... C.. ... ... ... ...
(3)... ... ... ... ... ..G ... ..A ..A ... ... ... ... ... ... ... ...
(4)..A ... ... ... ... ... ..G .G. ..A ... ... ... C.. ... ... ... ...

Des bactéries hébergeant les susdits clones DG 199 et DG 307 ont été déposées à la Collection Nationale des Cultures de Microorganismes de l'Institut Pasteur de Paris (CNCM), le 22 juillet 1986 respectivement sous les numéros I-580 et I-581. Des bactéries DG 145 ont été déposées le 15 septembre 1986 sous le numéro I-606.

## Revendications

1. Molécule, ou composition polypeptidique, comportant au moins une séquence peptidique porteuse d'un, ou plusieurs, épitope caractéristique d'une protéine produite dans les hépatocytes infectés par P. falciparum, caractérisée en ce que cette séquence peptidique est représentée par la formule : 
Leu-Ala-Lys-Glu-Lys-Leu-Gln-X-Gln-Gln-Ser-Asp-Leu-Glu-
Gln-Glu-Arg
 dans laquelle :
X est "Glu" (séquence peptidique I), ou "Gly" (séquence peptidique II).

2. Molécule selon la revendication 1, caractérisée en ce qu'elle est constituée d'un peptide monomère contenant 17 acides aminés, ce peptide contenant l'une ou l'autre des deux susdites séquences I et II.

3. Molécule selon la revendication 1, ou la revendication 2, caractérisée en ce qu'elle est constituée par un peptide de 17 acides aminés dont la structure correspond à l'une ou l'autre des deux susdites séquences I et II.

4. Molécule selon la revendication 1, caractérisée en ce qu'elle contient plusieurs séquences peptidiques répétitives correspondant à l'une ou l'autre seulement, ou aux deux séquences I et II sus-indiquées.

5. Molécule dérivée de celle selon l'une quelconque des revendications 1 à 5, par l'intermédiaire d'une permutation circulaire, notamment choisie parmi : 
Ala-Lys-Glu-Lys-Leu-Gln-X-Gln-Gln-Ser-Asp-Leu-Glu-Gln-
Glu-Arg-Leu

Lys-Glu-Lys-Leu-Gln-X-Gln-Gln-Ser-Asp-Leu-Glu-Gln-Glu-
Arg-Leu-Ala
 
Glu-Lys-Leu-Gln-X-Gln-Gln-Ser-Asp-Leu-Glu-Gln-Glu-Arg-
Leu-Ala-Lys

Lys-Leu-Gln-X-Gln-Gln-Ser-Asp-Leu-Glu-Gln-Glu-Arg-Leu-
Ala-Lys-Glu

Leu-Gln-X-Gln-Gln-Ser-Asp-Leu-Glu-Gln-Glu-Arg-Leu-Ala-
Lys-Glu-Lys

Gln-X-Gln-Gln-Ser-Asp-Leu-Glu-Gln-Glu-Arg-Leu-Ala-Lys-
Glu-Lys-Leu

X-Gln-Gln-Ser-Asp-Leu-Glu-Gln-Glu-Arg-Leu-Ala-Lys-Glu-
Lys-Leu-Gln

Gln-Gln-Ser-Asp-Leu-Glu-Gln-Glu-Arg-Leu-Ala-Lys-Glu-Lys-
Leu-Gln-X-

Gln-Ser-Asp-Leu-Glu-Gln-Glu-Arg-Leu-Ala-Lys-Glu-Lys-Leu-
Gln-X-Gln

Ser-Asp-Leu-Glu-Gln-Glu-Arg-Leu-Ala-Lys-Glu-Lys-Leu-Gln-
X-Gln-Gln-

Asp-Leu-Glu-Gln-Glu-Arg-Leu-Ala-Lys-Glu-Lys-Leu-Gln-X-
Gln-Gln-Ser-

Leu-Glu-Gln-Glu-Arg-Leu-ala-Lys-Glu-Lys-Leu-Gln-X-Gln-
Gln-Ser-Asp

Glu-Gln-Glu-Arg-Leu-Ala-Lys-Glu-Lys-Leu-Gln-X-Gln-Gln-
Ser-Asp-Leu

Gln-Glu-Arg-Leu-Ala-Lys-Glu-Lys-Leu-Gln-X-Gln-Gln-Ser-
Asp-Leu-Glu
 
Glu-Arg-Leu-Ala-Lys-Glu-Lys-Leu-Gln-X-Gln-Gln-Ser-Asp-
Leu-Glu-Gln

Arg-Leu-Ala-Lys-Glu-Lys-Leu-Gln-X-Gln-Gln-Ser-Asp-Leu-
Glu-Gln-Leu
 dans lesquels X est un résidu Glu ou Gly

6. Molécule selon l'une quelconque des revendications 1 à 5, caractérisée en ce qu'elle est constituée par un oligomère d'une ou des deux séquences peptidiques I et II sus-indiquées.

7. Molécule selon l'une quelconques des revendications 1 à 6, caractérisée en ce qu'elle est fixée à un support.

8. Composition immunogène caractérisée par l'association d'une molécule conforme à l'une quelconque des revendications 1 à 7, en association avec un véhicule pharmaceutiquement acceptable.

9. Composition de vaccin dirigée contre le paludisme, contenant entre autres principes immunogènes, une molécule conforme à l'une quelconque des revendications 1 à 7.

10. Séquence de nucléotides codant pour une ou l'autre des séquences peptidiques I et II définies dans la revendication 1.

11. Méthode de diagnostic in vitro du paludisme chez un individu susceptible d'être infecté par P. falciparum qui comprend la mise en contact d'un tissu ou d'un fluide biologique prélevé chez un individu avec une composition polypeptidique selon les revendications 1 à 10 dans des conditions permettant une réaction immunologique in vitro entre ladite composition polypeptidique et les anticorps éventuellement présents dans le tissu biologique, et la détection in vitro du complexe antigène-anticorps éventuellement formé.

12. Coffret ou kit pour le diagnostic in vitro du paludisme chez un individu susceptible d'être infecté par P. falciparum qui comprend :
- une composition polypeptidique selon les revendications 1 à 10,
- les réactifs pour la constitution du milieu propice à la réalisation de la réaction immunologique,
- les réactifs permettant la détection du complexe antigènes-anticorps produit par la réaction immunologique, de tels réactifs pouvant également porter un marqueur, ou être susceptibles d'être reconnus à leur tour par un réactif marqué, plus particulièrement dans le cas où la composition polypeptidique sus-mentionnée n'est pas marquée.

13. Les anticorps, polyclonaux ou monoclonaux, qui reconnaissent spécifiquement les séquences peptidiques selon l'une quelconque des revendications 1 à 5.

14. Les ADNs codant pour les polypeptides selon l'une quelconque des revendications 1 à 5.

15. Les ADNs recombinants contenant l'un au moins des ADNs de la revendication 14.

## Claims

1. Molecule, or peptide composition, containing at least one peptide sequence bearing one or more epitope(s) characteristic of a protein produced in hepatocytes infected by P.falciparum characterized in that this peptide sequence is represented by the formula: 
Leu-Ala-Lys-Glu-Lys-Leu-Gln-X-Gln-Gln-Ser-Asp-Leu-Glu-
Gln-Glu-Arg
 in which:
X is "Glu" (peptide sequence I), or "Gly" (peptide sequence II).

2. Molecule according to Claim 1, characterized in that it is constituted by a peptide monomer containing 17 amino acids, this peptide containing one or other of the two sequences I and II mentioned above.

3. Molecule according to Claim 1 or Claim 2, characterized in that it is constituted by a peptide of 17 amino acids, the structure of which corresponds to one or other of the two sequences I and II mentioned above.

4. Molecule according to Claim 1, characterized in that it contains several repetitive peptide sequences corresponding to one or other only, or to both of the sequences I and II indicated above.

5. Molecule derived from that conforming to any one of the Claims 1 to 5 through the intermediary of a circular permutation , selected in particular from : 
Ala-Lys-Glu-Lys-Leu-Gln-X-Gln-Gln-Ser-Asp-Leu-Glu-Gln-
Glu-Arg-Leu

Lys-Glu-Lys-Leu-Gln-X-Gln-Gln-Ser-Asp-Leu-Glu-Gln-Glu-
Arg-Leu-Ala

Glu-Lys-Leu-Gln-X-Gln-Gln-Ser-Asp-Leu-Glu-Gln-Glu-Arg-
Leu-Ala-Lys

Lys-Leu-Gln-X-Gln-Gln-Ser-Asp-Leu-Glu-Gln-Glu-Arg-Leu-
Ala-Lys-Glu

Leu-Gln-X-Gln-Gln-Ser-Asp-Leu-Glu-Gln-Glu-Arg-Leu-Ala-
Lys-Glu-Lys

Gln-X-Gln-Gln-Ser-Asp-Leu-Glu-Gln-Glu-Arg-Leu-Ala-Lys-
Glu-Lys-Leu
 
X-Gln-Gln-Ser-Asp-Leu-Glu-Gln-Glu-Arg-Leu-Ala-Lys-Glu-
Lys-Leu-Gln

Gln-Gln-Ser-Asp-Leu-Glu-Gln-Glu-Arg-Leu-Ala-Lys-Glu-Lys-
Leu-Gln-X-

Gln-Ser-Asp-Leu-Glu-Gln-Glu-Arg-Leu-Ala-Lys-Glu-Lys-Leu-
Gln-X-Gln

Ser-Asp-Leu-Glu-Gln-Glu-Arg-Leu-Ala-Lys-Glu-Lys-Leu-Gln-
X-Gln-Gln-

Asp-Leu-Glu-Gln-Glu-Arg-Leu-Ala-Lys-Glu-Lys-Leu-Gln-X-
Gln-Gln-Ser-

Leu-Glu-Gln-Glu-Arg-Leu-ala-Lys-Glu-Lys-Leu-Gln-X-Gln-
Gln-Ser-Asp

Glu-Gln-Glu-Arg-Leu-Ala Lys-Glu-Lys-Leu-Gln-X-Gln-Gln-
Ser-Asp-Leu

Gln-Glu-Arg-Leu-Ala-Lys-Glu-Lys-Leu-Gln-X-Gln-Gln-Ser-
Asp-Leu-Glu

Glu-Arg-Leu-Ala-Lys-Glu-Lys-Leu-Gln-X-Gln-Gln-Ser-Asp-
Leu-Glu-Gln

Arg-Leu-Ala-Lys-Glu-Lys-Leu-Gln-X-Gln-Gln-Ser-Asp-Leu-
Glu-Gln-Leu
 in which X is a Glu or Gly residue.

6. Molecule according to any one of the Claims 1 to 5, characterized in that it is constituted by an oligomer of one or both peptide sequences I and II indicated above.

7. Molecule according to any one of the Claims 1 to 6, characterized in that it is bound to a support.

8. Immunogenic composition characterized by the combination of molecule conforming to any one of the Claims 1 to 7 with a pharmaceutically acceptable vehicle.

9. Vaccinating composition directed against malaria containing, among other immunogenic principles, a molecule conforming to any one of the Claims 1 to 7.

10. Sequence of nucleotides coding for one or other of the peptides sequences I and II defined in Claim 1.

11. In vitro diagnostic method for malaria in a individual likely to be infected by P. falciparum which comprises the placing of a tissue or a biological fluid taken from a individual in contact with a polypeptide composition according to Claims 1 to 10 under conditions allowing an immunological reaction to take place in vitro between the said polypeptide composition and the antibodies possibly present in the biological tissue, and the in vitro detection of the antigen-antibody complex possibly formed.

12. Kit for the in vitro diagnosis of malaria in a individual likely to be infected by P. falciparum which contains :
- a polypeptide composition according to the Claims 1 to 10,
- the reagents for the constitution of an appropriate medium for carrying out the immunological reaction,
- the reagents making possible the detection of the antigen-antibody complex produced by the immunological reaction, whereby such reagents may also bear a marker, or be capable of being recognized in turn by a labelled reagent, more particularly when the above-mentioned peptide composition is not labelled.

13. The monoclonal or polyclonal antibodies which specifically recognize the peptide sequences according to any one of the claims 1 to 5.

14. The DNAs coding for the polypeptides according to any one of the Claims 1 to 5.

15. The recombinant DNAs containing at least one of the DNAs of Claim 14.

## Patentansprüche

1. Polypeptidmolekül oder -mittel, das mindestens eine Peptidsequenz enthält, die ein oder mehrere Epitop(e) trägt, welches/welche für ein in den durch P. falciparum infizierten Leberzellen erzeugtes Protein charakteristisch ist/sind, dadurch gekennzeichnet, daß diese Peptidsequenz durch die Formel 
-
Leu-Ala-Lys-Glu-Lys-Leu-Gln-X-Gln-Gln-Ser-Asp-Leu-Glu-
Gln-Glu-Arg
 wiedergegeben wird, in der
X "Glu" (Peptidsequenz I) oder "Gly" (Peptidsequenz II) ist.

2. Molekül nach Anspruch 1, dadurch gekennzeichnet, daß es aus einem monomeren Peptid besteht, das 17 Aminosäuren enthält, wobei dieses Peptid eine der beiden oben genannten Sequenzen I und II enthält.

3. Molekül nach Anspruch 1 oder Anspruch 2, dadurch gekennzeichnet, daß es aus einem Peptid von 17 Aminosäuren besteht und dessen Struktur einer der beiden oben genannten Sequenzen I und II entspricht.

4. Molekül nach Anspruch 1, dadurch gekennzeichnet, daß es mehrere sich wiederholende Peptidsequenzen enthält, die nur der einen oder anderen oder beiden oben genannten Sequenzen I und II entsprechen.

5. Molekül, abgeleitet von einem Molekül nach einem der Ansprüche 1 bis 5 durch eine intermediäre zirkulare Permutation, vor allem ausgewählt aus: 
Ala-Lys-Glu-Lys-Leu-Gln-X-Gln-Gln-Ser-Asp-Leu-Glu-Gln-
Glu-Arg-Leu

Lys-Glu-Lys-Leu-Gln-X-Gln-Gln-Ser-Asp-Leu-Glu-Gln-Glu-
Arg-Leu-Ala
 
Glu-Lys-Leu-Gln-X-Gln-Gln-Ser-Asp-Leu-Glu-Gln-Glu-Arg-
Leu-Ala-Lys

Lys-Leu-Gln-X-Gln-Gln-Ser-Asp-Leu-Glu-Gln-Glu-Arg-Leu-
Ala-Lys-Glu

Leu-Gln-X-Gln-Gln-Ser-Asp-Leu-Glu-Gln-Glu-Arg-Leu-Ala-
Lys-Glu-Lys

Gln-X-Gln-Gln-Ser-Asp-Leu-Glu-Gln-Glu-Arg-Leu-Ala-Lys-
Glu-Lys-Leu

X-Gln-Gln-Ser-Asp-Leu-Glu-Gln-Glu-Arg-Leu-Ala-Lys-Glu-
Lys-Leu-Gln

Gln-Gln-Ser-Asp-Leu-Glu-Gln-Glu-Arg-Leu-Ala-Lys-Glu-Lys-
Leu-Gln-X-

Gln-Ser-Asp-Leu-Glu-Gln-Glu-Arg-Leu-Ala-Lys-Glu-Lys-Leu-
Gln-X-Gln

Ser-Asp-Leu-Glu-Gln-Glu-Arg-Leu-Ala-Lys-Glu-Lys-Leu-Gln-
X-Gln-Gln-

Asp-Leu-Glu-Gln-Glu-Arg-Leu-Ala-Lys-Glu-Lys-Leu-Gln-X-
Gln-Gln-Ser-

Leu-Glu-Gln-Glu-Arg-Leu-ala-Lys-Glu-Lys-Leu-Gln-X-Gln-
Gln-Ser-Asp

Glu-Gln-Glu-Arg-Leu-Ala Lys-Glu-Lys-Leu-Gln-X-Gln-Gln-
Ser-Asp-Leu

Gln-Glu-Arg-Leu-Ala-Lys-Glu-Lys-Leu-Gln-X-Gln-Gln-Ser-
Asp-Leu-Glu
 
Glu-Arg-Leu-Ala-Lys-Glu-Lys-Leu-Gln-X-Gln-Gln-Ser-Asp-
Leu-Glu-Gln

Arg-Leu-Ala-Lys-Glu-Lys-Leu-Gln-X-Gln-Gln-Ser-Asp-Leu-
Glu-Gln-Leu
 worin X ein Rest Glu oder Gly ist.

6. Molekül nach einem der Ansprüche 1 bis 5, dadurch gekennzeichnet, daß es aus einem Oligomeren einer oder beider oben genannten Peptidsequenzen I und II besteht.

7. Molekül nach einem der Ansprüche 1 bis 6, dadurch gekennzeichnet, daß es auf einem Träger fixiert ist.

8. Immunogenes Mittel, charakterisiert durch die Kombination eines Moleküls gemäß einem der Ansprüche 1 bis 7 mit einem pharmazeutisch verträglichen Träger.

9. Impfmittel gegen Malaria, das unter anderen immunogenen Bestandteilen ein Molekül gemäß einem der Ansprüche 1 bis 7 enthält.

10. Nukleotidsequenz, die eine der beiden in Anspruch 1 definierten Peptidsequenzen I und II codiert.

11. In vitro-Diagnoseverfahren für Malaria bei einer Person, die von P. falciparum infiziert werden kann, dadurch gekennzeichnet, daß man ein Gewebe oder eine Körperflüssigkeit, entnommen bei/von der Person, mit einem Polypeptidmittel nach den Ansprüchen 1 bis 10 unter Bedingungen in Berührung bringt, die eine immunologische in vitro-Reaktion zwischen dem Polypeptidmittel und den gegebenenfalls im Körpergewebe vorhandenen Antikörpern ermöglichen, und in vitro den gegebenenfalls gebildeten Antigen-Antikörperkomplex nachweist.

12. Kleiner Koffer oder Kit für die in vitro-Diagnose von Malaria bei einer Person, die vom P. falciparum infiziert werden kann, der umfaßt:
- ein Polypeptidmittel nach den Ansprüchen 1 bis 10,
- Reaktionspartner für die Herstellung des Mediums, das für die Durchführung der immunologischen Reaktion geeignet ist,
- Reaktionspartner zum Nachweis des Antigen-Antikörper-Komplexes, der von der immunologischen Reaktion erzeugt wird, wobei diese Reaktionspartner auch einen Marker tragen können oder von einem markierten Reaktionspartner erkannt werden können, vor allem dann, wenn das oben genannte Polypeptidmittel nicht markiert ist.

13. Polyklonale oder monoklonale Antikörper, die spezifisch die Peptidsequenzen nach einem der Ansprüche 1 bis 5 erkennen.

14. Die DNAs, die für die Polypeptide nach einem der Ansprüche 1 bis 5 codieren.

15. Die rekombinierten DNAs, die mindestens eine DNA nach Anspruch 14 enthalten.
